# EUROPÄISCHE PATENTANMELDUNG

(11) **EP 1 342 712 A2**
(43) Veröffentlichungstag der Anmeldung: **10.09.2003**
(21) Anmeldenummer: 03002347.7
(22) Anmeldetag: 04.02.2003
(51) Int. Cl.: C07C 45/50, C07C 47/225

(54) **Verfahren zur Herstellung von Terpinolenaldehyd und seine Verwendung zur Herstellung von Riechstoffen**

(30) Priorität: 13.02.2002 DE 10205835
(71) Anmelder: Celanese Chemicals Europe GmbH, 60439 Frankfurt am Main (DE)
(72) Erfinder: Schmid, Klaus, Dr., 46539 Dinslaken (DE); Hoff, Dietmar, 42277 Wuppertal (DE)

(57) **Zusammenfassung**

Die Erfindung betrifft ein Verfahren zur Herstellung von Terpinolenaldehyd aus Terpinolen durch Hydroformylierungsreaktion bei Temperaturen von 100 bis 180°C und Drücken von 15 bis 35 MPa in Gegenwart einer Rhodium und Organophosphorverbindungen enthaltenden Komplexverbindung. Die Erfindungs betrifft ebenfalls die Herstellung von Terpinolenaldehyd aus Limonen, wobei in einer ersten Reaktionsstufe Limonen in Gegenwart einer wäßrigen Lösung, wasserlösliche organische Phosphor(III)verbindungen in komplexer Bindung enthaltender Übergangsmetallverbindungen der Gruppe VIII des Periodensystems in ein Gemisch aus Terpinolen und Limonenaldehyd umgewandelt wird, das anschließend in einer zweiten Reaktionsstufe nach den Bedingungen der Hydroformylierung von Terpinolen weiter umgesetzt wird.

## Beschreibung

Die Erfindung betrifft ein verbessertes Verfahren zur Herstellung von Terpinolenaldehyd [2-Formyl-p-menth-4(8)en] und seine Verwendung zur Herstellung von Folgeprodukten für die Riechstoffindustrie.

In der Parfümerie wurden anfangs ausschließlich natürliche Stoffe als Geruchsträger verwendet. Diese Stoffe stehen jedoch nicht immer in ausreichender Menge und in gleichbleibender Qualität zur Verfügung. Häufig ist ihre Verwendung auch aus ökologischen Gründen nur eingeschränkt möglich und überdies sind sie oft sehr teuer, so dass sich ihr Einsatz als Geruchsgeber für Massenprodukte verbietet. Daher werden heutzutage in großem Umfang synthetische Substanzen als Riechstoffe eingesetzt.

In den letzten Jahren haben neben einer großen Anzahl anderer Verbindungsklassen cyclische Acetale und Ketale als industriell erzeugte Riechstoffe an Bedeutung gewonnen. In einer umfangreichen Arbeit in Angew. Chemie 2000, 112, 3107-3138 wird der Einsatz von cyclischen Acetalen und Ketalen als Ambra-Riechstoffe beschrieben. Beispiele für derartige Verbindungen sind u.a. Acetale und Ketale, die sich von 1-Formyl-2,4-dimethylcyclohexen-3 und 2-Methyl-2-sec.butyl-propan-1,3-diol und von 2-Formylcaran und Aceton ableiten. Ferner werden Dioxolane und 1,3-Dioxane als potentielle Riechstoffe erwähnt. Diese Verbindungen sind aus α-Cedren bzw. aus Campher leicht zugänglich.

In der EP 0 276 998 A2 werden Parfümkompositionen beschrieben, die als Riechstoff mindestens eine Verbindung der allgemeinen Formel 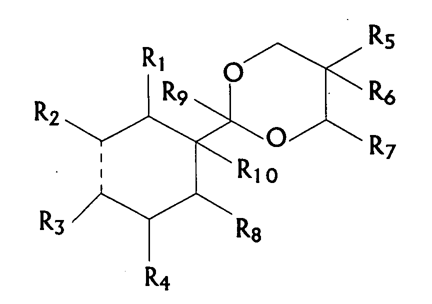 enthalten. In dieser Formel steht die gestrichelte Linie für eine Doppel- oder Einfachbindung innerhalb des Cyclohexanringes. R₁, R₂, R₃ und R₄ bedeuten Wasserstoff oder einen Methylrest. R₅ und R₆ sind Wasserstoff oder ein Alkyl-, ein Cycloalkyl- oder ein Alkenylrest mit 1 bis 4 Kohlenstoffatomen oder ein, gegebenenfalls substituierter, Ring, der bis zu 5 Kohlenstoffatome enthält. R₅ und R₆ können zusammen mit dem Kohlenstoffatom des Dioxanringes einen, gegebenenfalls substituierten, Ring mit bis zu 6 Kohlenstoffatomen bilden. R₇ bedeutet Wasserstoff, Methyl, Ethyl, n-Propyl, Isopropyl, n-Butyl, sec.Butyl, Isobutyl oder t-Butyl. R₈ und R₉ stehen jeweils für Wasserstoff oder den Methylrest. R₁₀ ist Wasserstoff, Methyl oder Isopropyl. Die Gesamtzahl der Kohlenstoffatome liegt im Bereich von 13 bis 20.

Die Herstellung der genannten Verbindungen erfolgt in einer ersten Stufe durch Diels-Alder-Reaktion von Dienen mit ungesättigten Aldehyden oder Ketonen, die zu cyclischen Carbonylverbindungen führt. Beispiele für geeignete Diene sind Butadien-1,3 und 2-Methyl-1,3-pentadien. Als Aldehyde oder Ketone kommen z.B. Acrolein, Methacrolein, Crotonaldehyd oder Methylvinylketon in Betracht. In einer zweiten Stufe werden die Carbonylverbindungen mit Diolen wie 2-Methyl-2-propyl-1,3-propandiol, 2-Methyl-2-sec.butyl-1,3-propandiol oder 2-Ethylhexan-1,3-diol zum erwünschten Acetal oder Ketal umgesetzt.

Bei weiteren Riechstoffen basierend auf cyclischen Acetalen leitet sich die Aldehydkomponente von ein- oder mehrfachsubstituierten Propionaldehyden ab, die als Substituenten in 3-Stellung eine, gegebenfalls substituierte, Cyclohexyl- oder Cyclohexenylgruppe enthalten. Riechstoffe auf Basis cyclischer Acetale, die sich von Diolen als Alkohol- und von substituierten Propionaldehyden als Aldehydkomponente ableiten lassen, sind Gegenstand der noch nicht veröffentlichten deutschen Patentanmeldung mit dem Aktenzeichen 101 58 907.7.

Die ein- oder mehrsubstituierten Propionaldehyde werden in vielen Fällen durch Hydroformylierung (Oxosynthese) aus olefinisch ungesättigten Vorstufen gewonnen. Als besonders wichtige Formylverbindung hat Limonenaldehyd (9-Formyl-p-menth-1-en) eine weite Verwendung gefunden. Es ist bekannt, Limonenaldehyd durch Hydroformylierung von Limonen [p-Mentha-1,8(9)dien] herzustellen. Gemäß der Offenlegungsschrift DE-2 849 742 arbeitet man dabei in Gegenwart von Rhodium und Triphenylphosphin als Komplexligand unter hohem Druck bei 10 bis 40 MPa, wobei sich die Formylgruppe an die exo-cyclische Doppelbindung anlagert. Dabei wird über eine Ausbeute von 83 % der Theorie berichtet. Die Offenlegungsschrift DE-2 849 742 weist darauf hin, dass unter den angewandten Hydroformylierungsbedingungen die Isomerisierung der in den Ausgangsolefinen vorhandenen Doppelbindungen weitgehend unterdrückt werden kann.

Führt man die Hydroformylierungsreaktion von Limonen gemäß der Lehre aus der deutschen Offenlegungsschrift DE-2 849 742 durch und untersucht man das erhaltene Hydroformylierungsgemisch, so läßt sich ein geringer Gehalt an Terpinolenaldehyd [2-Formyl-p-menth-4(8)en bzw. 2-Methyl-5(1-methylethyliden)cyclohexancarboxaldehyd] nachweisen. In geringem Maße findet unter den bekannten Hydroformylierungsbedingungen eine Isomerisierung des Limonens [p-Mentha-1,8(9)dien] in das Terpinolen [p-Mentha-1,4(8)dien] unter Verschiebung der endständigen in die innenständige exocyclische Doppelbindung statt. Diese innenständige exocyclische Doppelbindung verhält sich in Hydroformylierungsreaktionen sehr reaktionsträge und unter Hydroformylierung der methylsubstituierten Doppelbindung innerhalb des Cyclohexenrings kommt es bei der Hydroformylierung von Limonen in geringem Maße zur Bildung von Terpinolenaldehyd.

Im Terpinolenaldehyd ist die Formylgruppe am Terpenringgerüst gebunden und das der Formylgruppe benachbarte Kohlenstoffatom des Terpenrings trägt eine Methylgruppe. Es liegen ähnliche Strukturmerkmale vor, wie sie auch im 3-Formylpinan zu finden sind. Nach US 4,424,378 kann man 3-Formylpinan als interessante Ausgangssubstanz für die Herstellung von Produkten einsetzen, die als Aromastoff oder Geschmacksverstärker im Pharma-, Lebensmittel- oder Parfümbereich eine weite Anwendung finden.

Der breiten Verwendung von Terpinolenaldehyd als Ausgangskomponente für Riechstoffe und weiterer Anwendungen steht die bisherige schlechte und aufwendige Zugänglichkeit entgegen. So wird, wie bereits erwähnt, Terpinolenaldehyd bei der Hydroformylierung des leicht zugänglichen und preislich günstig verfügbaren Limonens nur in untergeordnetem Maße gebildet und muß unter hohem Aufwand isoliert werden. Auch die direkte Hydroformylierung von Terpinolen ergibt den gewünschten Terpinolenaldehyd nur in geringen Ausbeuten. Nach Flavour Fragrance J. 1991, 6(3), 179-82 führt man die Hydroformylierung von Terpinolen bei niedrigen Temperaturen (65°C) und geringem Druck (4,4 MPa) bei hohen Rhodiumkonzentrationen (11500 ppm) in sehr großer Verdünnung (Anteil des Lösugsmittels Benzol 73 Gew.-%, bezogen auf das Reaktionsgemisch) durch. Man wählt milde Reaktionsbedingungen, um Nebenreaktionen, wie die Isomerisierung des Terpinolens in Limonen oder die Hydrierung zu gesättigten Kohlenwasserstoffen und Alkoholen möglichst zu vermeiden. Als Folge der gewählten milden Reaktionsbedingungen hat man allerdings lange Reaktionszeiten in Kauf zu nehmen. Trotz der milden Reaktionsbedingungen wird bei einem Umsatz von 87% nur eine Selektivität von 28,7% zu dem gewünschten Terpinolenaldehyd beobachtet. Dabei beträgt die Reaktionszeit 96 Stunden.

Die bekannten Verfahren erfüllen in wirtschaftlicher und technischer Hinsicht noch nicht alle Anforderungen, die an einen im technischen Maßstab ausgeübten Prozeß gestellt werden, sei es, dass die Ausgangsstoffe nicht in ausreichender Menge und/oder nicht kostengünstig zur Verfügung stehen oder dass die Umwandlung der Ausgangsstoffe in den Aldehyd mit bisher nicht befriedigenden Ausbeuten erfolgt.

Es bestand daher die Aufgabe, ein Verfahren zu entwickeln, das in technisch einfacher Weise Terpinolen mit hoher Selektivität und hohen Ausbeuten in Terpinolenaldehyd überführt. Eine weitere Aufgabe bestand darin, ein Verfahren zur Herstellung von Terpinolenaldehyd bereitzustellen, das, ausgehend von preiswert verfügbaren Rohstoffen, in technisch einfacher Weise in hohen Ausbeuten Terpinolenaldehyd liefert.

Diese Aufgabe wird gelöst durch die Bereitstellung eines Verfahrens zur Herstellung von Terpinolenaldehyd aus Terpinolen. Es ist dadurch gekennzeichnet, dass man Terpinolen bei Temperaturen von 100 bis 180°C und Drücken von 15 bis 35 MPa in Gegenwart einer Rhodium und Organophosphorverbindung enthaltenden Komplexverbindung und gegebenenfalls überschüssiger Organophosphorverbindung mit Synthesegas, gegebenenfalls in Gegenwart eines organischen Lösungsmittels, umsetzt.

Diese Aufgabe wird ebenfalls gelöst durch die Bereitstellung eines Verfahrens zur Herstellung von Terpinolenaldehyd ausgehend von Limonen. Es ist dadurch gekennzeichnet, dass man Limonen in einer ersten Reaktionsstufe in einem heterogenen Reaktionssystem unter Verwendung einer wäßrigen Lösung, wasserlösliche organische Phosphor(III)verbindungen in komplexer Bindung enthaltender Übergangsmetallverbindungen der Gruppe VIII des Periodensystems der Elemente bei Temperaturen von 80 bis 140°C und Drücken von 1 bis 4 MPa in Gegenwart von Synthesegas behandelt, danach die organische Phase von der wäßrigen Phase trennt und anschließend die organische Phase in einer zweiten Reaktionsstufe bei Temperaturen von 100 bis 180°C und Drücken von 15 bis 35 MPa in Gegenwart einer Rhodium und Organophosphorverbindung enthaltenden Komplexverbindung und gegebenenfalls überschüssiger Organophosphorverbindung mit Synthesegas, gegebenenfalls in Gegenwart eines organischen Lösungsmittels, umsetzt.

Führt man die Hydroformylierung von Terpinolen gemäß der erfindungsgemäßen Arbeitsweise gegenüber dem Stand der Technik bei deutlich höheren Druck- und Temperaturbedingungen durch, kann vorteilhafterweise die eingesetzte Menge des teuren Rhodiums deutlich reduziert und die Reaktionszeit merklich vermindert werden. Überraschenderweise wird trotz der höheren Druck- und Temperaturbedingungen der gewünschte Terpinolenaldehyd mit einer deutlich besseren Ausbeute erhalten als nach dem aus dem Stand der Technik bekannten Prozeß. So liegt die Ausbeute an Terpinolenaldehyd nach dem erfindungsgemäßen Verfahren bei über 35 %, während nach dem Stand der Technik Ausbeuten um nur 25 % berichtet werden. Dies ist um so überraschender, da man bei der Wahl höherer Druckund Temperaturbedingungen verstärkt mit Nebenreaktionen, wie der Isomerisierung von Terpinolen in Limonen und der anschließenden Bildung von Limonenaldehyd, hätte rechnen müssen.

Die Hydroformylierungsreaktion von Terpinolen führt man in einem homogenen Reaktionssystem durch. Der Begriff homogenes Reaktionssytem steht für eine im wesentlichen aus Ausgangsolefin, Reaktionsprodukte und Katalysator zusammengesetzte homogene Lösung. Als Nebenprodukte sind in der homogenen Lösung durch Isomerisierung von Terpinolen Limonen und sein Hydroformylierungsprodukt Limonenaldehyd enthalten.

Ein Lösungsmittelzusatz ist nicht unbedingt erforderlich, er ist jedoch nicht auszuschließen. Führt man die Hydroformylierungsreaktion unter Lösungsmittelzusatz durch, so werden als Lösungsmittel organische Verbindungen eingesetzt, in denen Ausgangsmaterial, Reaktionsprodukt und Katalysatorsystem löslich sind. Beispiele für geeignete Lösungsmittel sind aromatische Kohlenwasserstoffe, wie Benzol und Toluol oder die Xylole. Andere gebräuchliche Lösungsmittel sind Paraffinöl, Cyclohexan, n-Hexan, n-Heptan oder n-Octan, Ether, wie Tetrahydrofuran, Ketone oder Texanol® der Firma Eastman. Der Anteil des Lösungsmittels im Reaktionsmedium kann über einen weiten Bereich variiert werden und beträgt üblicherweise zwischen 20 und 90 Gew.-%, vorzugsweise 50 bis 80 Gew.-%, bezogen auf das Reaktionsgemisch.

Als Katalysatoren werden Rhodium-Komplexverbindungen verwendet, die organische Phosphor(III)-Verbindungen als Liganden enthalten. Derartige Komplexverbindungen und ihre Herstellung sind bekannt (z.B. aus US-A-3 527 809, US-A-4 148 830, US-A-4 247 486, US-A-4 283 562). Sie können als einheitliche Komplexverbindungen oder auch als Gemisch unterschiedlicher Komplexverbindungen eingesetzt werden. Die Rhodium-Konzentration im Reaktionsmedium erstreckt sich über einen Bereich von etwa 10 bis etwa 1000 Gew.-ppm und beträgt vorzugsweise 10 bis 700 Gew.-ppm. Inbesondere wendet man Rhodium in Konzentrationen von 10 bis 50 Gew.-ppm, jeweils bezogen auf das homogene Reaktionsgemisch an. Als Katalysator kann die stöchiometrisch zusammengesetzte Rhodium-Komplexverbindung Anwendung finden. Es hat sich jedoch als zweckmäßig erwiesen, die Hydroformylierung in Gegenwart eines Katalysatorsystems aus Rhodium-Phosphor-Komplexverbindung und freiem, d.h. überschüssigen Phosphorliganden durchzuführen, der mit Rhodium keine Komplexverbindung mehr eingeht. Der freie Phosphorligand kann der gleiche sein, wie in der Rhodium-Komplexverbindung, es können aber auch von diesem verschiedene Liganden eingesetzt werden. Der freie Ligand kann eine einheitliche Verbindung sein oder aus einem Gemisch verschiedener Organophosphorverbindungen bestehen. Beispiele für Rhodium-Phosphor-Komplexverbindungen, die als Katalysatoren Anwendung finden können, sind in US-A-3 527 809 beschrieben. Zu den bevorzugten Liganden in den Rhodium-Komplexkatalysatoren zählen z. B. Triarylphosphine wie Triphenylphosphin, Trialkylphosphine wie Tri(n-octyl)phosphin, Trilaurylphosphin, Tri(cyclohexyl)phosphin, Alkylarylphosphine, Cycloalkylphenylphosphine, Alkylphosphite, Arylphosphite beispielsweise Tris-(2,4-di-tert.-butyl)phenylphosphit, Alkyldiphosphite oder Aryldiphosphite. Wegen seiner leichten Zugänglichkeit wird Triphenylphosphin besonders häufig angewandt.

Üblicherweise beträgt das molare Verhältnis von Rhodium zu Phosphor 1 : 1 bis 1 : 1000, jedoch kann der molare Anteil des Phosphors in Form organischer Phosphorverbindungen auch höher sein. Vorzugsweise setzt man Rhodium und organisch gebundenen Phosphor in molaren Verhältnissen von 1 : 30 bis 1 : 500 ein. Bei der Anwendung von Triarylphoshinen haben sich insbesondere Rhodium zu Phosphor-Molverhältnisse von 1 : 50 bis 1 : 100 bewährt.

Man führt die Hydroformylierungsreaktion bei erhöhten Temperaturen und Drücken durch. Im allgemeinen liegt der Druck in einem Bereich zwischen 10 und 35 MPa, vorzugsweise in einem Bereich von 25 bis 35 MPa. Im allgemeinen wählt man Temperaturen zwischen 100 und 180°C. Bevorzugt hält man Temperaturen von 120 bis 160 °C und insbesondere von 130 bis 140°C ein. Das molare Verhältnis von Wasserstoff zu Kohlenmonoxid im Synthesegas bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet.

Man bildet den Katalysator üblicherweise aus den Komponenten Rhodium oder Rhodiumverbindung, organische Phosphorverbindung und Synthesegas unter den Bedingungen der Hydroformylierungsreaktion im Reaktionsgemisch. Es ist aber auch möglich, den Katalysator zunächst zu präformieren und ihn anschließend der eigentlichen Hydroformylierungsstufe zuzuführen. Die Bedingungen der Präformierung entsprechen dabei im allgemeinen den Hydroformylierungsbedingungen.

Rhodium gelangt entweder als Metall oder als Verbindung zum Einsatz. Im metallischer Form verwendet man es entweder als feinverteilte Partikel oder in dünner Schicht auf einem Träger, wie Aktivkohle, Calciumcarbonat, Aluminiumsilikat, Tonerde niedergeschlagen. Als Rhodiumverbindungen eignen sich Salze aliphatischer Mono- und Polycarbonsäuren, wie Rhodium-2-ethylhexanoat, Rhodiumacetat, Rhodiumoxalat, Rhodiumpropionat oder Rhodiummalonat. Weiterhin können Rhodiumsalze anorganischer Wasserstoffund Sauerstoffsäuren, wie Rhodiumnitrat oder Rhodiumsulfat, die verschiedenen Rhodiumoxide oder auch Rhodiumcarbonylverbindungen wie Rh₃(CO)₁₂ oder Rh₆(CO)₁₆ oder Komplexverbindungen des Rhodiums, z.B. Cyclopentadienylrhodiumverbindungen oder Rhodiumacetylacetonat, eingesetzt werden. Rhodiumhalogenverbindungen kommen wegen ihres korrosiven Verhaltens der Halogenidionen weniger in Betracht.

Bevorzugt werden Rhodiumoxid und insbesondere Rhodiumacetat und Rhodium-2-ethylhexanoat.

Die Reaktion kann entweder absatzweise oder kontinuierlich durchgeführt werden.

Zweckmäßigerweise werden die Reaktionsbedingungen so eingestellt, daß bei Teilumsatz Terpinolenaldehyd mit möglichst hoher Selektivität gebildet wird. Bei der destillativen Aufarbeitung des Reaktionsgemisches kann nicht umgesetztes Terpinolen über den Kopf der Destillationskolonne abgezogen und in den Prozeß zurückgeführt werden während man den gewünschten Terpinolenaldehyd über einen Seitenabzug gewinnt. Der nach Abtrennung des Olefins und Aldehyds verbleibende, den Katalysator enthaltende Rückstand wird, gegebenenfalls nach Zusatz von Frischkatalysator und Entnahme eines Teils der im Verlauf der Reaktion gebildeten Hochsieder, in die Reaktionszone zurückgeführt.

Je nach Reinheitsanforderungen an Terpinolenaldehyd kann sich nach der Seitenabzugentnahme eine Behandlung des Terpinolenaldehyds mit Formaldehyd anschließen. Diese aus der DE 29 21 619 bekannte Methylenierung führt zu einer Kondensationsreaktion von α-unverzweigten Aldehyden, wie z.B. Limonenaldehyd, die noch in Restmengen im abgezogenen Terpinolenaldehyd enthalten sein können. Durch die Reaktion mit Formaldehyd werden Restmengen an α-unverzweigten Aldehyden in höhersiedende Kondensationsprodukte überführt, von denen Terpinolenaldehyd in höherer Reinheit auf einfache Weise destillativ abgetrennt werden kann. Formaldehyd wird in einer solchen Menge zugegeben, dass ein vollständiger Umsatz von α-unverzweigten Aldehyden, wie z.B. Limonenaldehyd, gewährleistet wird. Formaldehyd wird im allgemeinen bis zu einem Überschuß von 5%, bezogen auf die molare Menge an α-unverzweigten Aldehyden, eingesetzt.

Anstelle von Terpinolen als Ausgangsprodukt kann man auch das kostengünstigere und einfacher verfügbare Limonen einsetzten. Bei dieser Ausführungsform der Erfindung wird zunächst in einer ersten Reaktionsstufe Limonen teilweise zu Terpinolen in Gegenwart einer wäßrigen Katalysatorlösung und in Gegenwart von Synthesegas isomerisiert. Als wasserlösliche Katalysatoren setzt man organische Phosphor(III)-Verbindungen in komplexer Bindung enthaltende Übergangsmetallverbindungen der Gruppe VIII des Periodensystem der Elemente ein. Vorzugsweise verwendet man Komplexverbindungen von Kobalt, Rhodium, Nickel, Palladium, Platin, Eisen oder Ruthenium. Als besonders wirksame Katalysatoren haben sich wasserlösliche Komplexverbindungen des Rhodiums, Eisens, Platins oder Palladiums erwiesen.

Die Isomerisierung des Limonens in Terpinolen ist aus J. Mol. Cat. A: Chemical (2000), 152(1-2), 15-24 bekannt. Das bekannte Verfahren arbeitet mit homogen gelösten Isomerisierungskatalysatoren auf Basis von PtCl₂(PPh₃)₂ - SnCl₂ bei einer Temperatur von 130°C und einem Druck von 9 MPa über einen Zeitraum von 50 Stunden, wobei über eine Isomerisierungsrate von 70% berichtet wird. Das molare Verhältnis von Platin zu Phosphor zu Zinn in dem Isomerisierungskatalysator bewegt sich in einem Bereich von 1:4:1 bis 1:4:5. Bei dem bekannten Verfahren wird das teure Übergangsmetall mit einer Konzentration von 14300 ppm, bezogen auf das Olefin, eingesetzt.

In Gegensatz zu der bekannten Verfahrensvariante ist der erfindungsgemäße Isomerisierungsschritt durch das Vorliegen einer organischen Phase, die das olefinische Ausgangsmaterial und das Reaktionsprodukt enthält, und einer wäßrigen Phase, in der der Katalysator gelöst ist, charakterisiert. Nach erfolgter Umsetzung können die organische Phase und Katalysatorphase durch einfache Phasentrennung ohne thermische Belastung von einander getrennt werden und die wäßrige Katalysatorphase kann in den Prozeß zurückgeschleust werden. Eine derartige Reaktionsführung in einem Zweiphasensystem, die man auch als heterogene Reaktionsführung bezeichnet, ist z.B. in der DE-B-26 27 354 beschrieben. Danach werden Hydroformylierungsreaktionen olefinisch ungesättigter Verbindungen in Gegenwart wäßriger Katalysatorlösungen durchgeführt.

Als Katalysatoren für die Isomerisierung von Limonen werden wasserlösliche Komplexverbindungen der Übergangsmetalle der Gruppe VIII des Periodensystems der Elemente eingesetzt, die wasserlösliche organischen Phosphor(III)-Verbindungen als Liganden enthalten. Beispiele für wasserlösliche Phosphor(III)-Verbindungen, die mit den Übergangsmetallen Komplexverbindungen bilden, sind Triarylphosphine, Trialkylphosphine, gemischte aliphatische-aromatische Phosphine und arylierte bzw. alkylierte Diphosphine, deren organische Reste Sulfonsäuregruppen oder Carboxylgruppen enthalten. Ihre Herstellung und Anwendung ist z.B. aus DE-B 26 27 354, EP-B1-0 103 810, EP-B1-0 163 234 und EP-A1-0 571 819 bekannt. Weitere Gruppen geeigneter Verbindungen sind sulfonierte oder carboxylierte organische Phosphite oder Diphosphite sowie heterocyclische Verbindungen des dreiwertigen Phosphors, die z.B. aus EP-A1-0 575 785 und EP-A1-0 646 588 bekannt sind.

Die Bedingungen, unter denen die Isomerisierung in der ersten Reaktionsstufe abläuft, können innerhalb weiter Grenzen varrieren und den individuellen Gegebenheiten angepaßt werden. Üblicherweise führt man die Isomerisierung des Limonens bei Temperaturen von 80 bis 140°C durch. Bevorzugt hält man Temperaturen von 110 bis 120°C ein. Der Gesamtdruck erstreckt sich über einen Bereich von 1 bis 4 MPa, vorzugsweise von 1,5 bis 2,5 MPa. Das molare Verhältnis von Wasserstoff zu Kohlenmonoxid im Synthesegas bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet. Unter den angewandten Reaktionsbedingungen kommt es während der in der ersten Stufe ablaufenden Isomerisierung in gewissem Ausmaße zur Limonenaldehydbildung. Durch Isomerisierung gebildetes Terpinolen reagiert unter den Reaktionsbedingungen des Zweiphasenprozesses nur in einem untergeordneten Maße unter Aldehydbildung ab.

Die Konzentration an dem Übergangsmetall beträgt 50 bis 5000 Gew.-ppm, vorzugsweise 250 bis 2000 Gew.-ppm und insbesondere 500 bis 1000 Gew.ppm, jeweils bezogen auf das eingesetzte Olefin. Obgleich es möglich ist, als Katalysator die stöchiometrisch zusammengesetzte Übergangsmetall-Phosphor-Komplexverbindung einzusetzen, arbeitet man üblicherweise in Gegenwart von überschüssigem Phosphorliganden, d.h. Ligand, der mit dem Übergangsmetall keine komplexe Bindung eingegangen ist. Je mol des jeweiligen Übergangsmetalls wendet man bevorzugt 1 bis 100 mol Phosphor in Form einer wasserlöslichen organischen Phosphor(III)verbindung an. Besonders bewährt haben sich molare Verhältnisse von Übergangsmetall zu Phosphor im Bereich von 1:10 bis 1:100. Der Übergangsmetall-Phosphor-Komplexkatalysator braucht nicht einheitlich zusammengesetzt sein, sondern kann z.B. aus einem Gemisch von Übergangsmetall-Komplexverbindungen bestehen, die sich durch die Art der Phosphorliganden unterscheiden. Ebenso kann der in der wäßrigen Katalysatorlösung enthaltene freie Phosphorligand aus einem Gemisch unterschiedlicher wasserlöslicher organischer Phosphor(III)verbindungen zusammengesetzt sein. Man bildet den Katalysator üblicherweise aus den Komponenten Übergangsmetall oder Übergangsmetallverbindung, wasserlösliche, organische Phosphor(III)verbindung und Synthesegas unter den Bedingungen der Isomerisierungssreaktion im Reaktionsgemisch. Es ist aber auch möglich, den Katalysator zunächst zu präformieren und ihn anschließend der eigentlichen Isomerisierungsstufe zuzuführen. Die Bedingungen der Präformierung entsprechen dabei im allgemeinen den Isomerisierungsbedingungen.

Das Übergangsmetall gelangt entweder als Metall oder als Verbindung zum Einsatz. Im metallischer Form verwendet man es entweder als feinverteilte Partikel oder in dünner Schicht auf einem Träger, wie Aktivkohle, Calciumcarbonat, Aluminiumsilikat, Tonerde niedergeschlagen. Als Übergangsmetallverbindungen eignen sich Salze aliphatischer Mono- und Polycarbonsäuren, wie 2-Ethylhexanoate, Acetate, Oxalate, Propionate oder Malonate. Weiterhin können auch Salze anorganischer Wasserstoff- und Sauerstoffsäuren, wie Nitrate oder Sulfate, die verschiedenen Übergangsmetalloxide oder auch Übergangsmetallcarbonyle oder Übergangsmetallkomplexverbindungen eingesetzt werden.

Als bevorzugtes Übergangsmetall wird Rhodium eingesetzt und geeignete Rhodiumverbindungen sind beispielsweise Rh₃(CO)₁₂ oder Rh₆(CO)₁₆ oder Cyclopentadienylrhodiumverbindungen oder Rhodiumacetylacetonat. Bevorzugt werden Rhodiumoxid und insbesondere Rhodiumacetat und Rhodium-2-ethylhexanoat.

Um die Isomerisierungsreaktion je Zeiteinheit von olefinisch ungesättigten Verbindungen, die in der wäßrigen Katalysatorlösung nur wenig löslich sind, zu erhöhen, kann es sich empfehlen, dieser Lösung ein Phasentransferreagenz (Lösungsvermittler) zuzusetzen. Er verändert die physikalischen Eigenschaften der Grenzflächen zwischen den beiden flüssigen Phasen und erleichtert den Übergang des organischen Reaktanten in die wäßrige Katalysatorlösung.

Als Lösungsvermittler sind Verbindungen bekannt, deren hydrophile Gruppen ionisch (anionisch oder kationisch) oder nicht ionisch sind. Zu den anionenaktiven Verbindungen gehören Natrium-, Kalium- oder Ammoniumsalze von Carbonsäuren, vorzugsweise solcher mit 8 bis 20 Kohlenstoffatomen und insbesondere von gesättigten Fettsäuren mit 12 bis 18 Kohlenstoffatomen, ferner Alkylsulfate, Alkylbenzolsulfonate und Alkylbenzolphosphate. Beispiele für kationische Lösungsvermittler sind Tetraalkylammoniumund N-Alkylpyridiniumsalze. Die nichtionischen Phasentransferreagenzien dissoziieren in wäßriger Lösung nicht in Ionen. Zu ihnen zählen Alkylpolyethylenglykole, Alkylphenylpolyethylenglykole, Fettsäurealkylolamine und Trialkylaminoxide. Ferner finden Ampholyte wie Aminocarbonsäuren, Betaine und Sulfobetain als Lösungsvermittler Anwendung. Entsprechende Verfahren sind z.B. aus EP-B1-0 157 316 bekannt.

Als nichtionische Phasentransferreagenzien verwendet man beispielsweise Polyethylenglykole bis zu einem Gehalt von 40 Gew.-%, vorzugsweise in einem Bereich von 15 bis 35 Gew.%, jeweils bezogen auf die wäßrige Katalysatorphase. Als Polyethylenglykole eignen sich beispielsweise PEG400 oder PEG750. Hierbei charakterisiert die Zahlenangabe die durchschnittliche Molmasse des Polyethylenglykols.

Die Isomerisierung kann sowohl absatzweise als auch kontinuierlich durchgeführt werden. Im allgemeinen wird die Isomerisierungsreaktion des Limonens bis zu einer Terpinolenanreicherung von bis zu 20 Gew.-%, bezogen auf das Reaktionsgemisch, gefahren, um überlange Reaktionszeiten zu vermeiden. Nach beendeter Reaktion wird das Reaktionsprodukt der Isomerisierungsreaktion in einem Phasentrenner von der wäßrigen Katalysatorlösung, die in den Prozeß zurückgeführt wird, getrennt.

Das organische Reaktionsprodukt wird anschließend ohne weitere Reinigung in einer zweiten Reaktionsstufe in Gegenwart von Rhodium mit Synthesegas in einem homogenen Reaktionssystem umgesetzt. Die Hydroformylierungsbedingungen in der zweiten Reaktionsstufe entsprechen dabei den Bedingungen der Terpinolenhydroformylierung.

Als Ergebnis des erfindungsgemäßen zweistufigen Verfahrens wird ein Aldehydgemisch erhalten, in dem der Terpinolenaldehydgehalt auf bis zu 20 Gew.-%, bezogen auf das gesamte Reaktionsprodukt, angereichert ist. Als Hauptkomponente liegt Limonenaldehyd mit bis zu 80 Gew.-%, bezogen auf das gesamte Reaktionsgemisch, vor.

Die destillative Auftrennung des Reaktionsgemisches in die reinen Komponenten Limonenaldehyd und Terpinolenaldehyd kann auf konventionellem Wege erfolgen.

Es hat sich jedoch als nützlich erwiesen, bei der destillativen Aufarbeitung eines Limonenaldehyd und Terpinolenaldehyd enthaltenden Aldehydgemisches Formaldehyd hinzusetzen. Aus DE 29 216 19 ist die Methylenierung von Limonenaldehyd bekannt. Da Terpinolenaldehyd nur über ein reaktives Wasserstoffatom in der α-Stellung zur Carbonylgruppe verfügt, ist bei Terpinolenaldehyd eine analoge Reaktion mit Formaldehyd, wie sie beim Limonenaldehyd bekannt ist, unter Anlagerung einer Methylengruppe nicht möglich. Je nach der Menge des zugesetzten Formaldehyds enhält die rohe Aldehydmischung Anteile aus Limonenaldehyd, 2-Methylenlimonenaldehyd [2-Methylen-3-(4-methylcyclohex-3-enyl)-butanal] und Terpinolenaldehyd.

Im allgemeinen gibt man Formaldehyd in einer solchen Menge hinzu, dass ein vollständiger Umsatz der α-unverzweigten Aldehydverbindungen, also auch des Limonenaldehyds, gewährleistet wird. Die durch Methylenierung gebildeten Kondensationsprodukte weisen einen höheren Siedepunkt auf und von ihnen kann der niedriger siedende Terpinolenaldehyd, der nicht zur Methylenierungsreaktion mit Formaldehyd fähig ist, destillativ leichter abgetrennt werden. Auf diese Weise lassen sich Terpinolenaldehyd und 2-Methylenlimonenaldehyd als Reinsubstanzen gewinnen.

Gemische aus Limonenaldehyd und 2-Methylenlimonenaldehyd finden ebenfalls in der Riechstoffindustrie Verwendung. Nach der DE 198 20 657 kann durch Zusatz von Gemischen aus Limonenaldehyd und 2-Methylenlimonenaldehyd zu bereits bekannten Kompositionen eine Intensivierung, Abrundung und Haftung dieser Kompositionen erzielt werden.

Das erfindungsgemäße Verfahren eröffnet einen einfachen und kostengünstigen Weg zur Herstellung von Terpinolenaldehyd aus dem in großen Mengen verfügbaren Limonen. Als Koppelprodukt erhält man Limonenaldehyd oder, falls man das Hydroformylierungsgemisch mit Formaldehyd umsetzt, entsprechend 2-Methylenlimonenaldehyd, das im Gemisch mit Limonenaldehyd ebenfalls für die Riechstoffindustrie von Bedeutung ist. Je nach den wirtschaftlichen Gegebenheiten, wie der Verfügbarkeit des teuren Terpinolens läßt sich Terpinolenaldehyd entweder direkt durch Hydroformylierung von Terpinolen oder aber aus dem kostengünstig verfügbaren Limonen durch Isomerisierung und anschließender Hydroformylierung in wirtschaftlicher Weise gewinnen.

Aufgrund der hohen Reaktivität der Formylgruppe kann man Terpinolenaldehyd in eine Reihe von Folgeprodukten überführen, die als Riechstoffe, Aromastoffe oder als Geschmacksverstärker im Lebensmittelsektor Anwendung finden.

Die vorliegenden Beispiele sollen das erfindungsgemäße Verfahren näher erläutern, selbstverständlich ist das neue Verfahren nicht auf diese Ausführungsformen beschränkt.

### Beispiele:

### A: Herstellung von Terpinolenaldehyd aus Terpinolen

### Vergleichsbeispiel gemäß Flavour and Frangrance Journal, Vol. 6, 179-182 (1991)

In einem Autoklaven wurden 4,09 g Terpinolen zusammen mit 0,42 g Tris(triphenylphosphin)rhodiumcarbonylhydrid (entsprechend 50 mg Rhodium) und 0,36 g Triphenylphospin (Molverhältnis Rhodium zu Phosphor 1 zu 6) vorgelegt, mit 13,2 g Benzol verdünnt und anschließend 96 Stunden bei einer Temperatur von 65°C und einem Druck von 4,4 MPa hydroformyliert. Bei einem Umsatz von 87% wurde eine Selektivität von 28,7% an 2-Methyl-5-(1-methylethyliden)cyclohexancarboxaldehyd (Terpinolenaldehyd) erhalten.

### Beispiel 1

In einem 0,5I-Autoklav, ausgestattet mit magnetischer Rührung, wurden 300 g Terpinolen (Terpinolengehalt: 90%) zusammen mit 1,5 g einer toluolischen Lösung von Rhodium-2-ethylhexanoat (entsprechend 9 mg Rhodium) und 2,3 g Triphenylphospin, entsprechend einem Molverhältnis von Rhodium zu Phosphor von 1 zu 100, vorgelegt und anschließend 12 Stunden bei einer Temperatur von 130 °C und einem Druck von 26 MPa hydroformyliert.

Neben 135,5 g nicht umgesetztem Terpinolen wurden 99,4 g 2-Methyl-5-(1-methylethyliden)cyclohexancarboxaldehyd (Terpinolenaldehyd) erhalten. Der Umsatz an Terpinolen lag bei 49,8%, entsprechend einer Selektivität an Terpinolenaldehyd von 60,5%.
Siedepunkt 78-79°C/5 hPa; δ ¹³C: 203,08 ppm (CHO);
128,39 ppm, 122,53 ppm (C-4, C-8); 57,75 ppm; 32,10 ppm (CH-2, CH-1);
35,05 ppm, 29,21 ppm, 29,18 ppm (CH₂-3, CH₂-6, CH₂-5);
20,10 ppm, 20,00 ppm, 19,90 ppm (CH₃-7, CH₃-9, CH₃-10).
Meßgerät: Bruker ARX 400M; 100,6 MHz in C₆D₆.

### Beispiel 2

Gemäß Beispiel 1 wurden 75,6 g Terpinolen (Terpinolengehalt: 90%) zusammen mit 0,63 g einer toluolischen Lösung von Rhodium-2-ethylhexanoat (entsprechend 3,9 mg Rhodium) und 0,98 g Triphenylphospin, entsprechend einem Molverhältnis von Rhodium zu Phosphor von 1 zu 100, vorgelegt, mit 50 g Toluol verdünnt und anschließend 12 Stunden bei einer Temperatur von 130 °C und einem Druck von 26 MPa hydroformyliert.

Neben 28,6 g nicht umgesetztem Terpinolen wurden 32,7 g 2-Methyl-5-(1-methylethyliden)cyclohexancarboxaldehyd erhalten. Der Umsatz an Terpenolen lag bei 58,0%, entsprechend einer Selektivität an Terpenolenaldehyd von 67,9%.

### Beispiel 3

Gemäß Beispiel 1 wurden 75,6 g Terpinolen (Terpinolengehalt: 90%) zusammen mit 1,26 g einer toluolischen Lösung von Rhodium-2-ethylhexanoat (entsprechend 7,6 mg Rhodium) und 1,96 g Triphenylphospin, entsprechend einem Molverhältnis von Rhodium zu Phosphor von 1 : 102 vorgelegt, mit 50 g Toluol verdünnt und anschließend 12 Stunden bei einer Temperatur von 130 °C und einem Druck von 26 MPa hydroformyliert.

Neben 31,2 g nicht umgesetztem Terpinolen wurden 29,4 g 2-Methyl-5-(1-methylethyliden)cyclohexancarboxaldehyd erhalten. Der Umsatz an Terpinolen lag bei 54,2%, entsprechend einer Selektivität an Terpinolenaldehyd von 60,5%.

### Beispiel 4

Gemäß Beispiel 1 wurden 75,6 g Terpinolen (Terpinolengehalt: 90%) zusammen mit 0,63 g einer toluolischen Lösung von Rhodium-2-ethylhexanoat (entsprechend 3,9 mg Rhodium) und 0,98 g Triphenylphospin, entsprechend einem Molverhältnis von Rhodium zu Phpsphor von 1 zu 102 vorgelegt, mit 50 g Toluol verdünnt und anschließend 12 Stunden bei einer Temperatur von 140 °C und einem Druck von 26 MPa hydroformyliert.

Neben 24,7 g nicht umgesetztem Terpinolen wurden 31,9 g 2-Methyl-5-(1-methylethyliden)cyclohexancarboxaldehyd erhalten. Der Umsatz an Terpinolenaldehyd lag bei 63,8%, entsprechend einer Selektivität an Terpinolenaldehyd von 60,3%.

### Beispiel 5

Gemäß Beispiel 1 wurden 75,6 g Terpinolen (Terpinolengehalt: 90%) zusammen mit 0,63 g einer toluolischen Lösung von Rhodium-2-ethylhexanoat (entsprechend 3,9 mg Rhodium) und 0,98 g Triphenylphospin, entsprechend einem Molverhältnis von Rhodium zu Phosphor von 1 zu 102 vorgelegt und anschließend 12 Stunden bei einer Temperatur von 130 °C und einem Druck von 26 MPa hydroformyliert.

Neben 30,4 g nicht umgesetztem Terpinolen wurden 32,3 g 2-Methyl-5-(1-methylethyliden)cyclohexancarboxaldehyd erhalten. Der Umsatz an Terpinolen lag bei 55,3%, entsprechend einer Selektivität an Terpinolenaldehyd von 70,4%.

### B: Herstellung von Terpinolenaldehyd ausgehend von Limonen

### Vergleichsbeispiel 6 (entsprechend DE 28 49 742)

In einem 5I-Autoklav, ausgestattet mit magnetischer Rührung, wurden 2100 g Limonen (Limonengehalt: 95,4%) zusammen mit 10,55 g einer toluolischen Lösung von Rhodium-2-ethylhexanoat (entsprechend 630 mg Rhodium) und 16,06 g Triphenylphospin, entsprechend einem Rhodium zu Phosphor verhältnis von 1 zu 100 vorgelegt und anschließend 8 Stunden bei einer Temperatur von 130°C und einem Druck von 26 MPa hydroformyliert. Das erhaltene Oxo-Rohprodukt (2589,5 g) wurde einer Claisendestillation unterzogen. Bei einer Kopftemperatur von 93°C/4 mbar (4 hPa) gingen 2532,6 g eines farblosen Destillates über, das 2247,5 g Limonenaldehyd entsprechend einer Ausbeute von 91,9% enthielt. Als Nebenprodukte entstanden durch Isomerisierung 45,6 g Terpinolen (2,3%) und durch Hydroformylierung von Terpinolen auch 23,3 g 2-Methyl-5-(1-methylethyliden)cyclohexancarboxaldehyd (Terpinolenaldehyd), entsprechend einer Ausbeute von 1,0% bezogen auf eingesetztes Limonen.

### Beispiel 7:

In einem 5I-Autoklav, ausgestattet mit magnetischer Rührung, wurden 817,2 g Limonen (Limonengehalt: 95,4%) zusammen mit 0,62 g Rhodium in Form von Rhodiumacetat, 600 g PEG400 und 1162 g einer wässrigen Lösung von Trinatriumtri(m-sulfophenyl)phosphin (TPPTS-Lösung) (321 g NaTPPTS, entsprechend einem Rhodium zu Phosphorverhältnis von 1 zu 107) vorgelegt und anschließend 16 Stunden bei einer Temperatur von 125°C und einem Synthesegasdruck von 2 MPa behandelt. Das nach Phasentrennung erhaltene organische Rohprodukt (825,3 g) wies einen Terpinolengehalt von 15,9% auf, neben 58,1% Limonen und 11% Limonenaldehyd.

100 g eines so erhaltenen organischen Rohprodukts, enthaltend 13,3 g Limonenaldehyd, 62,0 g Limonen, 18 g Terpinolen und 6,7 g sonstige Komponenten wurde gemäß Beispiel 1 einer mit Triphenylphosphin modifizierten Hochdruckhydroformylierung unterzogen. Dabei wurde das Olefingemisch zusammen mit 0,69 g einer toluolischen Lösung von Rhodium-2-ethylhexanoat (entsprechend 4,3 mg Rhodium) und 1,1 g Triphenylphosphin, entsprechend einem Molverhältnis von Rhodium zu Phosphor von 1 zu 100, vorgelegt und anschließend 12 Stunden bei einer Temperatur von 130°C und einem Druck von 26 MPa hydroformyliert. Neben 9,2 g nicht umgesetztem Terpinolen und 87,1 g Limonenaldehyd wurden 10,1 g Terpinolenaldehyd (2-Methyl-5-(1-methylethyliden)cyclohexancarboxaldehyd) erhalten, entsprechend einem Gemisch enthaltend 74,8 Gew.-% Limonenaldehyd, 8,7 Gew.-% 2-Methyl-5-(1-methylethyliden)cyclohexancarboxaldehyd, 7,9 Gew.-% Terpinolen und 8,6 Gew.-% sonstige Komponenten, jeweils bezogen auf die gesamte Mischung.

## Patentansprüche

1. Verfahren zur Herstellung von Terpinolenaldehyd aus Terpinolen, **dadurch gekennzeichnet, dass** man Terpinolen bei Temperaturen von 100 bis 180°C und Drücken von 15 bis 35 MPa in Gegenwart einer Rhodium und Organophosphorverbindung enthaltenden Komplexverbindung und gegebenenfalls überschüssiger Organophosphorverbindung mit Synthesegas, gegebenenfalls in Gegenwart eines organischen Lösungsmittels, umsetzt.

2. Verfahren gemäß Anspruch 1 **dadurch gekennzeichnet, dass** man bei Drücken von 25 bis 35 MPa und bei Temperaturen von 120 bis 160°C arbeitet.

3. Verfahren gemäß Anspruch 1 oder 2 **dadurch gekennzeichnet, dass** man Rhodium in einer Konzentration von 10 bis 1000 Gew.-ppm, bezogen auf das homogene Reaktionsgemisch, einsetzt.

4. Verfahren nach Anspruch 3, **dadurch gekennzeichnet, dass** man Rhodium in einer Konzentration von 10 bis 700 Gew.-ppm, vorzugsweise 10 bis 50 Gew.-ppm, bezogen auf das homogene Reaktionsgemisch, einsetzt.

5. Verfahren nach einem oder mehreren der Ansprüche 1 bis 4, **dadurch gekennzeichnet, dass** das molare Verhältnis von Rhodium zu Phosphor 1:1 bis 1:1000 beträgt.

6. Verfahren nach Anspruch 5, **dadurch gekennzeichnet, dass** das molare Verhältnis von Rhodium zu Phosphor 1:30 bis 1:500, insbesondere 1:50 bis 1:100 beträgt.

7. Verfahren nach einem oder mehreren der Ansprüche 1 bis 6, **dadurch gekennzeichnet, dass** man als Organophosphorverbindungen Triarylphosphine, Trialkylphosphine, Alkylarylphosphine, Alkylphosphite, Arylphosphite, Alkyldiphosphite oder Aryldiphosphite verwendet.

8. Verfahren nach einem oder mehreren der Ansprüche 1 bis 7, **dadurch gekennzeichnet, daß** man das erhaltene Reaktionsprodukt nach destillativer Aufarbeitung mit Formaldehyd umsetzt.

9. Verfahren zur Herstellung von Terpinolenaldehyd ausgehend von Limonen, **dadurch gekennzeichnet, dass** man Limonen in einer ersten Reaktionsstufe in einem heterogenen Reaktionssystem unter Verwendung einer wäßrigen Lösung, wasserlösliche organische Phosphor(III)verbindungen in komplexer Bindung enthaltender Übergangsmetallverbindungen der Gruppe VIII des Periodensystems der Elemente bei Temperaturen von 80 bis 140°C und Drücken von 1 bis 4 MPa in Gegenwart von Synthesegas behandelt, danach die organische Phase von der wäßrigen Phase trennt und anschließend die organische Phase in einer zweiten Reaktionsstufe bei Temperaturen von 100 bis 180°C und Drücken von 15 bis 35 MPa in Gegenwart einer Rhodium und Organophosphorverbindung enthaltenden Komplexverbindung und gegebenenfalls überschüssiger Organophosphorverbindung mit Synthesegas, gegebenenfalls in Gegenwart eines organischen Lösungsmittels, umsetzt.

10. Verfahren nach Anspruch 9, **dadurch gekennzeichnet, dass** man in der ersten Reaktionsstufe bei einer Temperatur von 110 bis 120°C und bei Drücken von 1,5 bis 2,5 MPa arbeitet.

11. Verfahren nach Anspruch 9, **dadurch gekennzeichnet, dass** man in der ersten Reaktionsstufe als Übergangsmetallverbindungen der Gruppe VIII des Periodensystems der Elemente wasserlösliche Komplexverbindungen des Rhodiums, Eisens, Platins oder Palladiums einsetzt.

12. Verfahren nach Anspruch 9, **dadurch gekennzeichnet**, das man in der ersten Reaktionsstufe als wasserlösliche organische Phosphor(III)verbindungen Triarylphosphine, Trialkylphosphine, gemischte aliphatische-aromatische Phosphine, arylierte Diphosphine, alkylierte Diphosphine oder organische Phosphite und Diphosphite, deren organische Reste Sulfonsäuregruppen oder Carboxylgruppen enthalten, verwendet.

13. Verfahren nach einem oder mehreren der Ansprüche 9 bis 12, **dadurch gekennzeichnet, dass** in der ersten Reaktionsstufe die Konzentration an dem Übergangsmetall der Gruppe VIII des Periodensystems der Elemente 50 bis 5000 Gew.-ppm, bezogen auf eingesetztes Olefin, beträgt.

14. Verfahren nach Anspruch 13, **dadurch gekennzeichnet, dass** in der ersten Reaktionsstufe die Konzentration an dem Übergangsmetall der Gruppe VIII des Periodensystems der Elemente 250 bis 2000 Gew.-ppm, vorzugsweise 500 bis 1000 Gew.-ppm, jeweils bezogen auf das eingesetzte Olefin, beträgt.

15. Verfahren nach einem oder mehren der Ansprüche 9 bis 14, **dadurch gekennzeichnet, dass** in der ersten Reaktionsstufe je mol des Übergangsmetalls der Gruppe VIII des Periodensystems der Elemente 1 bis 100 mol Phosphor in Form der wasserlöslichen organischen Phosphor(III)verbindung verwendet wird.

16. Verfahren nach Anspruch 15, **dadurch gekennzeichnet, dass** in der ersten Reaktionsstufe das Molverhältnis zwischen dem Übergangsmetall der Gruppe VIII des Periodensystems der Elemente und Phosphor in Form der wasserlöslichen organischen Phosphor(III)verbindung 1:10 bis 1:100 beträgt.

17. Verfahren gemäß Anspruch 9, **dadurch gekennzeichnet, dass** man die zweite Reaktionsstufe gemäß einem oder mehreren der Ansprüche 2 bis 7 durchführt.

18. Verfahren zur Herstellung von Terpinolenaldehyd ausgehend von Limonen, **dadurch gekennzeichnet, dass** man das gemäß einem oder mehreren der Ansprüche 9-17 erhaltene Reaktionsprodukt mit Formaldehyd umsetzt und anschließend destilliert.

19. Verfahren nach Anspruch 18, **dadurch gekennzeichnet, dass** die Menge an Formaldehyd so bemessen ist, dass im Reaktionsprodukt enthaltener Limonenaldehyd teilweise oder vollständig in 2-Methylenlimonenaldehyd überführt wird.

20. Verwendung von Terpinolenaldehyd zur Herstellung von Folgeprodukten für Riechstoffe, Aromastoffe oder Geschmacksverstärker.
